# EUROPEAN PATENT APPLICATION

(11) **EP 1 698 648 A1**
(43) Date of publication of application: **06.09.2006**
(21) Application number: 05075496.9
(22) Date of filing: 01.03.2005
(51) Int. Cl.: C08G 14/12, C08G 14/04, C07C 69/732

(54) **Hydroxy-aromatic compound, process for the preparation thereof, and use of the compound**

(71) Applicant: DSM IP Assets B.V., 6411 TE Heerlen (NL)
(72) Inventor: Van Benthem, Rudolfus Antonius Theodorus Maria, 6141 BR Limbricht (NL); Kierkels, Renier Henricus Maria, 6099 AT Geleen (NL)
(74) Representative: Koster, Nico

(57) **Abstract**

The invention relates to a hydroxy-aromatic compound of formula (I)- 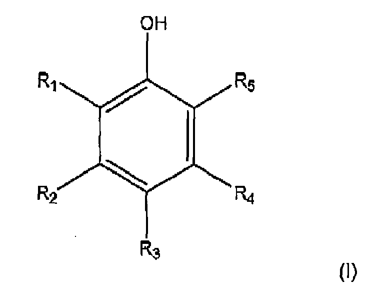 wherein:
■ at least one of the set consisting of R₁, R₃, and R₅ is a group of formula (II); any remaining one or two of the set consisting of R₁, R₃, and R₅ being H, OH, a C₁-C₁₂ alkyl group or an oligomeric or polymeric system;
■ R₂ and R₄ are H, OH, a C₁-C₁₂ alkyl group or an oligomeric or polymeric system;
■ formula (II) is the following group: 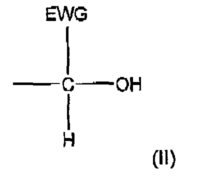
wherein EWG is an electron-withdrawing group.

## Description

The invention relates to a hydroxy-aromatic compound.

Hydroxy-aromatic compounds as such are known, and are defined as compounds having an aromatic ring with at least one -OH group attached directly to it. An example of such a compound is phenol. Another example of such a compound is the known adduct of phenol and formaldehyde; this hydroxy-aromatic compound is used in the preparation of phenol-formaldehyde resins. These resins are known from for example A. Knop, L.A. Pilato, Phenolic Resins, Springer Verlag Berlin 1990. These resins have many known uses, such as for example the use of these resins in adhesives for the preparation of particle boards.

A disadvantage of the known hydroxy-aromatic compounds, and in particular of their formaldehyde adducts, is that their use is associated with health risks, relating to the emission of formaldehyde during resin preparation, resin curing and in end products.

It is the objective of the present invention to reduce or even eliminate the said disadvantage while still providing a compound suitable for the preparation of hydroxy-aromatic resins.

The objective is achieved in that the hydroxy-aromatic compound is a compound of formula (I) 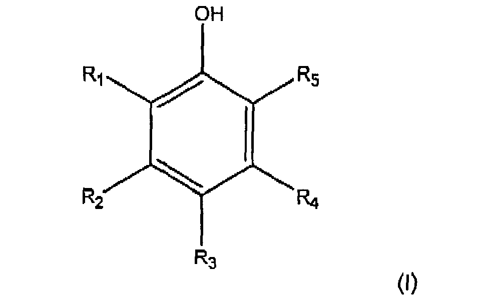 wherein:
- at least one of the set consisting of R₁, R₃, and R₅ is a group of formula (II); any remaining one or two of the set consisting of R₁, R₃, and R₅ being H, OH, a C₁-C₁₂ alkyl group or an oligomeric or polymeric system;
- R₂ and R₄ are H, OH, a C₁-C₁₂ alkyl group, or an oligomeric or polymeric system;
- formula (II) is the following group: 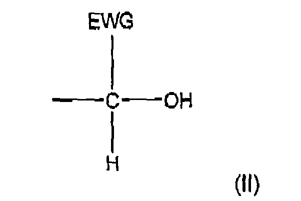
wherein EWG is an electron-withdrawing group.

The advantage of the compound according to the invention is that hydroxy-aromatic-based resins can be made that suffer less, or even not at all, from the health risks associated with the use of formaldehyde. Thus, resins prepared with the compound according to the present invention are in particular suitable for use in many applications such as adhesives, coatings, laminates, and shaped articles.

As is known in hydroxy-aromatic chemistry, the positions on the aromatic ring adjacent to and opposite the hydroxy group (i.e., ortho and para) have a different reactivity than the remaining two meta-positions. In formula (I), therefore, the groups R₁, R₃, and R₅ should be regarded within a similar context and are herein referred to as a set.

In the compound according to the invention, at least one of the groups in the set consisting of R₁, R₃, and R₆ is given by formula (II); the other one or two groups in the said set - in case not all three of the said set is given by formula (II) - is/are H, OH, or C₁-C₁₂ alkyl group, preferably H, OH, a C₁-C₉ alkyl group, or an oligomeric or polymeric system. If there are two groups not according to formula (II) then they may be the same or may be different. The oligomeric or polymeric system may be a hydroxy-aromatic resin, either of the resol or of the novolac type, preferably of the novolac type; or it may be a different type of thermosetting or thermoplastic system. Some examples of how the set according to the invention can be shaped are: R₁ is a group according to formula (II), R₃ is H, and R₅ is H; R₁ is a group according to formula (II), R₃ is H, and R₅ is CH₃; R₁ is H, R₃ is a group according to formula (II), and R₅ is H; R₁ and R₃ are a group according to formula (II), R₆ is H; R₁, R₃, and R₅ are all a group according to formula (II).

In the compound according to the invention, R₂ and R₄ are H, OH, a C₁-C₁₂ alkyl group, or an oligomeric or polymeric system; preferably R₂ and R₄ are H, OH or a C₁-C₉ alkyl group. R₂ and R₄ may be the same or may be different. Some preferred embodiments of R₂ and R₄ are: R₂ is OH and R₄ is H; R₂ is CH₃ and R₄ is H; R₂ is CH₃ and R₄ is CH₃; R₂ is H and R₄ is C₄H₉. R₁ and R₂ may be part of a multicyclic compound: the same holds mutatis mutandis for R₂ and R₃, R₃ and R₄, or R₄ and R₅.

The group according to formula (II) is an integral part of the compound according to the invention; it is either R₁, R₃, or R₅ in formula (I), or two of those, or all three. In formula (II), EWG is an electron-withdrawing group. EWG's are as such known to the skilled person. Examples of an EWG are acid-, ester-, cyano-, di-alkylacetal-, aldehyde-, substituted phenyl-, or trihalomethyl groups. Hydrogen is not an EWG. In a preferred embodiment, the group of formula (II) is a group according to formula (III): 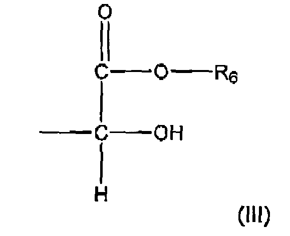 wherein R₆ is a C₁-C₁₂ alkyl group, aryl group, aralkyl group or cycloalkyl group. Preferably R₆ is a C₁-C₁₂ alkyl group; examples hereof are methyl, ethyl, propyl, butyl, pentyl, hexyl; more preferably, R₆ is a methyl group or an ethyl group.

In a preferred embodiment of the hydroxy-aromatic compound according to the invention, at least one of the set consisting of R₁, R₃, and R₅ is H. This has the advantage that the hydroxy-aromatic compound is better suitable for the preparation of the oligomeric or polymeric structures typical for hydroxy-aromatic resins. In another preferred embodiment, two of the set consisting of R₁, R₃, and R₅ are H. This has the advantage that such a hydroxy-aromatic compound can be used to create three-dimensional networks, an ability often desired in hydroxy-aromatic resins. The same ability of the hydroxy-aromatic compound to create three-dimensional networks is present in those embodiments where all of R₁, R₃, and R₅ are either H or a group according to formula (II).

The invention further relates to a process for the preparation of the hydroxy-aromatic compound as described above. The process according to the invention comprises a reaction step wherein a hydroxy-aromatic compound of formula (IV) is brought into contact with a compound according to formula (V), optionally in the presence of a catalyst, whereby formula (IV) is: 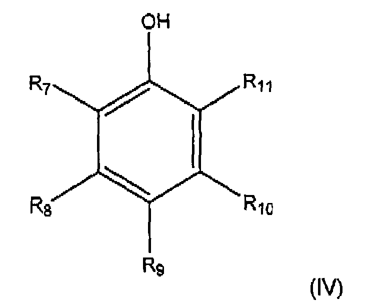 wherein R₇, R₆, R₉, R₁₀ and R₁₁ are H, OH, a C₁-C₁₂ alkyl group or an oligomeric or polymeric system, whereby at least one and preferably two or even three of the set consisting of R₇, R₉, and R₁₁ is or are H; and formula (V) is: 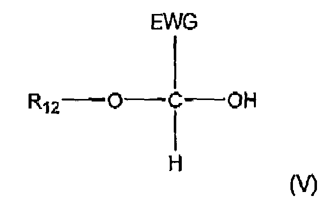 wherein EWH is an electron-withdrawing group and wherein R₁₂ is H, a C₁-C₁₂ alKyl group, aryl group, aralkyl group or cycloalkyl group.

Preferably, the compound according to formula (V) is an alkanol hemacetal according to formula (VI): 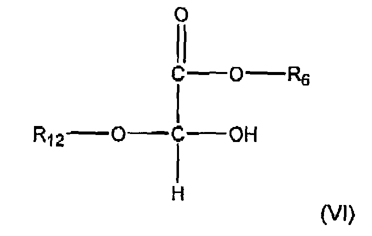 wherein R₆ is a C₁-C₁₂ alkyl group, aryl group, aralkyl group or cycloalkyl group and wherein R₁₂ is H, a C₁-C₁₂ alkyl group, aryl group, aralkyl group or cycloalkyl group. Preferably R₆ and R₁₂ are C₁-C₁₂ alkyl groups. Examples thereof are methyl, ethyl, propyl, butyl, pentyl, hexyl, and heptyl. R₆ and R₁₂ are in particular a methyl group or an ethyl group.

The process according to the invention comprises a reaction step. The purpose of the reaction step is to let a hydroxy-aromatic compound of formula (IV) react with a compound of formula (V). Thus these compounds must be brought together. The compound according to either formula (IV) or formula (V) may be one single compound or a mixture of two or more compounds falling within the scope of the formulas as defined above. Examples of preferred compounds according to formula (IV) are phenol, (2, 3, or 4-)cresol, resorcinol, (2, 3, or 4-)tert-butylphenol, (2, 3, or 4-)nonylphenol, (2,3- 2,4- 2,5- 2,6- or 3,4-)dimethylphenol, and (2, 3, or 4-)ethylphenol. Examples of compounds according to formula (V), in particular of the preferred alkanol hemiacetals according to formula (VI), are methylglyoxylate methanol hemiacetal (GMHA™, DSM Fine Chemicals, Linz); ethylglyoxylate ethanol hemiacetal (GEHA™, DSM Fine Chemicals, Linz); ethylglyoxylate methanol hemiacetal; butylglyoxylate butanol hemiacetal; butylglyoxylate methanol hemiacetal; butylglyoxylate ethanol hemiacetal; isopropylglyoxylate isopropanol hemiacetal; propylglyoxylate propanol hemiacetal; cyclohexylglyoxylate methanol hemiacetal and 2-ethylhexylglyoxylate methanol hemiacetal. Further examples of compounds according to formula V are glyoxylic acid hydrate, methylglyoxylate hydrate and ethylglyoxylate hydrate.

It may be beneficial to execute the reaction step according to the invention in a solvent or dispersant. As solvents, those compounds are suitable in which the reactants dissolve sufficiently to let the reaction take place. Examples of such solvents are water and various organic solvents. Depending on the specific compound or compounds of formula (IV) and (V), it may well be possible to use one or more of the reactants as solvent; in such a case, it can be possible to forego on the use of a solvent that is essentially a non-reactant and to execute the reaction step in bulk. In particular, many of the compounds according to formula (V) and in particular according to formula (VI) are a liquid at temperatures between 10°C and 100°C and can act as dispersant/solvent as well as reactant.

Although the reaction step may proceed spontaneously once the respective compounds have been brought together, it may be useful to bring the compounds together in the presence of a catalyst in order to accelerate the reaction. As catalyst, preferably an acid or a base is used; in particular, a Lewis or a Bronsted type of acid is preferred - such as for example sulphuric acid - whereby the pH is reduced to between 0 and 5, preferably to between 1 and 4, in particular to between 2 and 3. Suitable examples of acid catalysts are sulphuric acid, nitric acid, hydrochloric acid, phosphoric acid, boric acid, tetrafluoroboric acid, paratoluene sulphonic acid, formic acid, ammonium sulphate, ammonium chloride, ammonium nitrate. Suitable examples of basic catalysts are ammonia, trimethyl amine, triethyl amine, DABCO (diaza-bicyclo-octane), DBU (diaza-bicyclo-undecene), DMAP (4-dimethylaminopyridine), sodium hydroxide, potassium hydroxide.

The temperature in the reaction step of present process can vary within wide limits, and preferably lies between 10°C and 100°C. More preferably the process is carried out at between 40°C and 90°C. The pressure in the present process preferably is between 0.005 MPa and 1.0 MPa, preferably between 0.02 MPa and 0.2 MPa; most preferably, the pressure is atmospheric.

As consequence of the reaction step, a compound according to formula (I) is formed; additionally, the compound R₁₂OH is released as by-product. It may be desirable to isolate the compound according to formula (I); this may be achieved through techniques that are as such known, such as for example a combination of pH change, solvent exchange, evaporation and/or precipitation. If the compound according to formula (I) is not isolated, it may still be desirable to remove R₁₂OH; this may be achieved through techniques that are as such known, such as for example distillation. It may, however, also be acceptable or even desirable to let R₁₂OH remain in the presence of the compound according to formula (I).

In the process for the preparation of the hydroxy-aromatic compound according to the invention, the molar ratio between the EWG-containing compound according to formula (V) (E) and the hydroxy-aromatic compound according to formula (IV) (H), herein referred to as E/H ratio, may vary between wide limits. Preferably, the E/H ratio lies between about 0.1 and about 10, more preferably between about 0.5 and about 3. If the E/H ratio is about 0.5 or lower, the resulting hydroxy-aromatic compound according to the invention can be a mixture having a significant amount of a compound according to formula (I) in which one of the set consisting of R₁, R₃, and R₅ is a group of formula (II). If the E/H ratio is about 3 or higher, the resulting hydroxy-aromatic compound according to the invention can be a mixture having a significant amount of a compound according to formula (I) in which all three of the set consisting of R₁, R₃, and R₅ are a group of formula (II). If the E/H ratio is about 1 or 2, the resulting hydroxy-aromatic compound according to the invention can be a mixture in which compounds according to formula (I) in which one, two or all three of the set consisting of R₁, R₃, and R₅ are a group of formula (II) are ail clearly represented.

The invention further relates to a process for the preparation of a hydroxy-aromatic resin. Such processes are as such known and comprise condensation reactions between a hydroxy-aromatic compound and a compound such as an aldehyde, and typically also subsequent condensation reactions; an example of such a process is the process for preparation of a phenol-formaldehyde resin. In the process according to the invention, a compound according to formula (I) is used in the (subsequent) condensation reactions. The (subsequent) condensation reactions may be executed in the same fashion and under similar conditions as described above for the preparation of the compound according to formula (I). The compound falling within the scope of formula (V) and in particular formula (VI) may be - aside from the hydroxy-aromatic compound according to formula (I) and/or the already formed oligomeric or polymeric structures - the sole other compound participating in the condensation reactions in the resin; it may also be possible to use other compounds such as aldehdyes like formaldehyde or furfural (C₅H₄O₂) in combination with the compound according to formula (V). Preferably, however, at least 5 or 10 mol.% of the compounds participating in the condensation reactions with a hydroxy-aromatic moiety in the resin are one or more compounds according to formula (V); more preferably, this is at least 20 or 30%: in particular, this is at least 40 or 50%, with strong preference, at least 60 or 70 mol.% of the compounds reacting with a hydroxy-aromatic moiety in the resin are one or more compounds according to formula (V); most preferably, this is at least 80 or 90% or even essentially 100%.

The resin comprises hydroxy-aromatic compounds (H) - i.e. the compound as well as its condensation product. The resin also comprises EWG-containing compounds and possibly aldehyde compounds - i.e. the compound as well as its condensation product - together referred to as A. The resin thus has a molar A/H ratio. The molar A/H ratio in the resin preferably lies between 0.5 and 3, more preferably between 0.75 and 2. If the molar A/H ratio lies above 1, resol-type of resins can be formed whereby reactive 'A'-derived hydroxy groups are available. If the molar A/H ratio lies below 1, novolac-type of resins can be formed, in which essentially all 'A'-derived hydroxyl functionality has reacted away to form C-C and C-O ether bonds.

Resulting from the process as described above, the invention also relates to hydroxy-aromatic resins thus obtainable.

According to an embodiment of the invention, a hydroxy-aromatic resin can be prepared directly from raw materials comprising a compound according to formula (IV) as hydroxy-aromatic compound, and a compound according to formula (V). The conditions for achieving this are similar to those given above for the process or preparing the compound according to formula (I), and can be established by the skilled person via simple routine experimentation and using also his knowfedge of the preparation of phenol-formaldehyde resins.

The invention moreover relates to the use of the hydroxy-aromatic aldehyde resin according to the invention for the preparation of coatings or shaped articles such as particle boards, laminates, or mineral wool such as stone wool or glass wool. To this end, the resins may be used by methods and under conditions similar to those known per se from the use of known hydroxy-aromatic aldehyde resins like phenol-formaldehyde resins. A catalyst and other additives may be added to the resin before the resin is used for processing in its final application. Examples of customary additives are mould release agents, antistatic agents, adhesion promoters, plasticizers, colour enhancing agents, flame retardants, fillers, flow promoters, colorants, diluents, polymerization initiators, UV-stabilizers and heat stabilizers. Examples of fillers are glass fibres, mica, carbon fibres, metal fibres, clay, aramide fibres and strong polyethylene fibres.

The resin according to the invention may be used as such; however, it is also possible to subject the resin to a modification step; this is a reaction step designed to alter or enhance its functionality in a specific way. An example of an altered functionality is the solubility of the resin in water. An example of an enhanced functionality is the addition of a reactive group. An example of a modification step is the bringing of the resin in contact with compounds that react with the -OH groups; an example of such a compound is epichlorohydin. Another example of a modification step is the bringing of the resin in contact with compounds that react with the -OR₆ groups; an example of such a compound is water; the hydrolysis of the -OR₆ group into a -COOH group increases the solubility of the resin in water. Also, the modification step may be achieved through a transesterefication reaction between the -OR₆ groups and suitable compounds such as amines.

The present invention can be carried out in a variety of ways, as illustrated by the following non-limiting illustrative embodiments.

In a first illustrative embodiment, phenol and formaldehyde are used as raw materials to prepare - in a known fashion - a novolac-type of resin at a pH of 2. The phenol-formaldehyde novolac resin is then brought together with methylglyoxylate methanol hemiacetal (GMHA); this is done in an aqueous environment, at a temperature below 50°C and with an acidic catalyst at a pH of 2. The reaction is continued for 30 minutes, methanol being released and a resin according to the invention is formed. The resin according to the invention is then either used as adhesive in the production of a particle board or in the preparation of mineral wool. For the production of particle board, dosing, press factors and other circumstances are normal (eg a temperature of about 140°C). The resulting particle board will have a greatly reduced emission of formaldehyde compared to a particle board prepared by using only a standard phenol-formaldehyde resin. For the production of mineral wool such as stone wool, the resin is mixed with water so that a mixture having about 98% water content is formed. This mixture is then sprayed on stone wool as it is just formed and has a temperature of about 1900°C. The mixture acts as coolant, and at the same time the resin is cured thereby forming the end product; this all takes place in about 3 seconds.

In a second illustrative embodiment, phenol and GMHA are brought together in an aqueous solution. The molar ratio between phenol and GMHA is 1:2. At a slightly elevated temperature (below 50°C) and at pH of 2, a resol-type of resin will be formed in about 30 minutes. Then, the pH is raised to 5 by using NaOH and the temperature brought to room temperature so that a stable resin is obtained. Afterwards, the resin is brought ready for use by reducing the pH to 1. The resin is then used to prepare stone wool in the same fashion as in the previous illustrative embodiment.

In a third illustrative embodiment, cresol and GMHA are brought together in an aqueous solution. The molar ratio between cresol and GMHA is 4:3. Under conditions of elevated temperature (90°C) and reduced pH (2), a novolac-type of resin will be formed. This resin can be converted into a water-born coating by adding a hardener such as adipic acid dihydrazide (ADH). If ADH is used as hardener, the coating will cure quickly (within 30 minutes) and at room temperature.

In a fourth illustrative embodiment, cresol and GMHA are brought together in an aqueous solution. The molar ratio between cresol and GMHA is 4:3. Under conditions of elevated temperature (90°C) and reduced pH (2), a novolac-type of resin will be formed. The ester functionalities of the resin are then saponified by raising the pH to 10. Subsequently, a reduction of pH to about 2 and evaporation of the water will yield a acid-functional solid resin. This resin is then mixed with an epoxy resin such as Epikote™ 828 (a liquid epoxy resin produced from bisphenol A and epichlorohydrin). The resulting mixture is then moulded and pressed to cure in the form of a shaped article.

## Claims

1. Hydroxy-aromatic compound of formula (I): 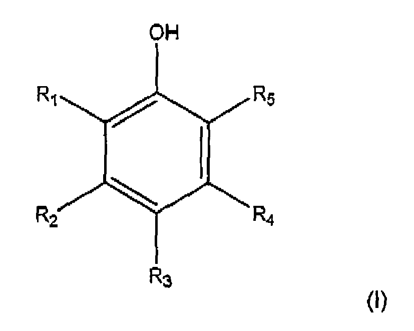 wherein:
• at least one of the set consisting of R₁, R₃, and R₅ is a group of formula (II); any remaining one or two of the set consisting of R₁, R₃, and R₅ being H, OH, a C₁-C₁₂ alkyl group or an oligomeric or polymeric system;
• R₂ and R₄ are H, OH, a C₁-C₁₂ alkyl group or an oligomeric or polymeric system;
• formula (II) is the following group: 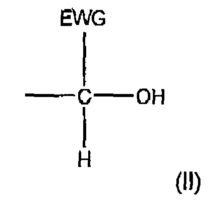
wherein EWG is an electron-withdrawing group.

2. Hydroxy-aromatic compound according to claim 1, wherein formula (II) is a group according to formula (III): 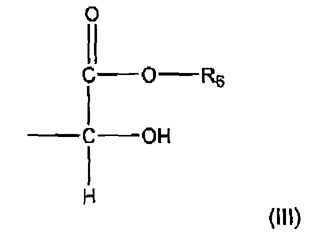 wherein R₆ is a C₁-C₁₂ alkyl group, aryl group, aralkyl group or cycloalkyl group.

3. Hydroxy-aromatic compound according to claim 1 or 2, wherein one or two of the set consisting of R₁, R₃, and R₅ is/are a group of formula (II) and wherein at least one of the said remaining one or two of the set consisting of R₁, R₃, and R₅ is H.

4. Process for the preparation of a hydroxy-aromatic compound according any one of claims 1-3, comprising a reaction step wherein a hydroxy-aromatic compound of formula (IV) is brought into contact with a compound according to formula (V), optionally in the presence of a catalyst, whereby formula (IV) is: 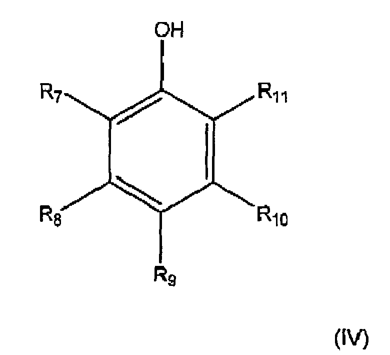 wherein R₇, R₈, R₉, R₁₀ and R₁₁ are H, OH, C₁-C₁₂ alkyl group, or an oligomeric or polymeric system, whereby at least one of the set consisting of R₇, R₉, and R₁₁ is H;
and formula (V) is: 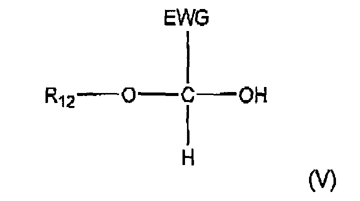 wherein R₁₂ is a C₁-C₁₂ alkyl group, aryl group, aralkyl group or cycloalkyl group.

5. Process according to claim 4, wherein the compound according to formula (V) is an alkanol hemiacetal according to formula (VI): 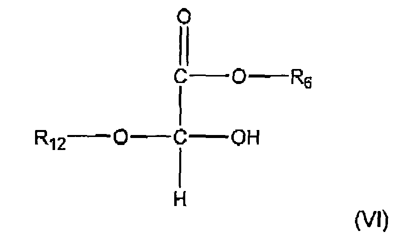 wherein R₆ and R₁₂ are as defined above.

6. Process according to claim 4 or 5, wherein the reaction step is carried out in water as solvent and wherein an acid is used as catalyst.

7. Process for the preparation of a hydroxy-aromatic resin, **characterized in that** a compound according to any one of claims 1-3 is used as a raw material.

8. Process for the preparation of a hydroxy-aromatic resin, **characterized in that** the raw materials comprise a compound according to formula (IV) as hydroxy-aromatic compound, and a compound according to formula (V).

9. Hydroxy-aromatic resin, obtainable by the process of claim 7 or 8

10. Use of the hydroxy-aromatic resin according to claim 9 in coatings or in the manufacture of shaped articles.
